# EUROPEAN PATENT APPLICATION

(11) **EP 3 892 668 A1**
(43) Date of publication of application: **13.10.2021**
(21) Application number: 20168531.0
(22) Date of filing: 07.04.2020
(51) Int. Cl.: C08G 69/20, C07D 263/08, C07D 263/32

(54) **LATENT CATALYST SYSTEMS AND PROCESSES FOR THE PRODUCTION OF POLYAMIDES**

(71) Applicant: Universität Stuttgart, 70174 Stuttgart (DE)
(72) Inventor: Buchmeiser, Michael, 73630 Remshalden (DE); Elser, Iris, 70569 Stuttgart (DE); Altmann, Hagen, 73540 Heubach-Lautern (DE); Benedikter, Mathis, 71384 Weinstadt (DE); Naumann, Stefan, 73733 Esslingen (DE)
(74) Representative: Held, Stephan

(57) **Abstract**

The invention concerns catalysts systems for the preparation of polyamides, in particular from lactam precursors, which comprise (i) a lactam activator (A) with increased electron deficiency at the intracyclic carbonyl carbon or precursors for the in-situ formation thereof and/or a Lewis acid (L) and (ii) a N-heterocyclic carbene. Corresponding catalysts systems provide for a latent polymerization activity towards lactams, which allows the formation of unreactive catalyst system/monomer mixtures, which can be polymerized by heating the mixture above an activation temperature. In addition, the inventive catalyst systems provide for fast reaction times at comparatively low temperatures and a high monomer conversion. The present invention further concerns processes for the production of polyamides, which employ such catalyst systems, polymerization systems comprising such catalyst systems and polyamide monomers and corresponding polyamides prepared from such process.

## Description

The invention concerns catalyst systems for the preparation of polyamides, in particular from lactam precusors, which comprise (i) a lactam activator (A) with increased electron deficiency at the intracyclic carbonyl carbon or precursors for the in-situ formation thereof and/or a Lewis acid (L) and (ii) a N-heterocyclic carbene. The present invention further concerns processes for the production of polyamides, which employ such catalyst systems, polymerization systems comprising such catalyst systems and polyamide monomers and corresponding polyamides prepared from such process.

### State of the art

The conversion of lactams into polyamides is a highly relevant commercial process, which today is performed in a million-ton scale worldwide. Important monomers are in particular ε-caprolactam (ε-CLA) and laurolactam, which can be reacted to PA6 or PA12.

The polymerization of these lactams can in principle be carried out by either cationic, hydrolytic or anionic polymerization. The latter two procedures are technically used. In the hydrolytic process, a defined amount of water (5-10%) is added and a portion of the monomer is hydrolyzed to the open-chain amino acid. Propagation then occurs by ring opening and by condensation of the amino acids. The reaction times are usually several hours (12-24 h) at temperatures well above 200 °C.

Anionic polymerization proceeds via the ring opening polymerization and has the advantage that it can be conducted at lower temperatures. As bases, for example, alkali metals, amides or Grignard compounds have been used. The choice of base is important: weak bases generate only low amount of lactam anions since the pKs of ε-CLA is 27.2 (in DMSO at 25 °C). On the other hand, strong bases can lead to side reactions, as e.g. protons of the CH-unit in the alpha-position to the carbonyl can be abstracted. The anionic polymerization proceeds via the so-called "activated monomer" (lactam-anion). Anionic polymerizations proceed very fast, typically within a few minutes. Often, isocyanates or N-acyl lactams are added as activators.

Polyamides can be prepared e.g. with the screw-injection molding technique (SIM) or as cast polyamides, e.g. by processing via reaction injection molding (RIM process). In the case of screw-injection molding, molten polymer is filled into a mold under pressure, whereas in reaction injection molding the monomeric melt is filled into a mold without pressure, before the polymerization is performed. Usually, injection molding processes are characterized by shorter tool running times. However, cast polyamides can be used for more complex components because the monomer melt shows a significant better flow behavior than the polymer melt (lower melting point, lower viscosity).

NHCs (= N-heterocyclic carbenes) are a class of substances with carbenes in a cyclic structure which contain at least one nitrogen atom (see e.g. Nature, 2014, 510, 485-496). In NHCs, the carbenoid carbon atom is predominantly sp₂-hybridized and in a singlet electron configuration in which the non-binding sp₂-orbital is occupied with two electrons and the p-orbital is unoccupied (Chem. Soc. Rev., 2013, 42, 6723-6753). The nitrogen atom might be covalently bound to an aliphatic or aromatic hydrocarbon. Ring sizes from three up to seven atoms have been described (Angew. Chem. Int. Ed., 2008, 47, 3122-3172; Polym. Chem., 2013, 4, 2731-2740). Most common NHCs are however five-membered rings, which contain two or three nitrogen atoms, such as imidazolidines, imidazolines and triazol based compounds. In these structures, a nitrogen atom might can also be substituted by i.a. oxygen, sulfur, a tetra-substituted carbon atom or phosphor.

The use carbenes as initiators/catalysts for the polymerization of lactams has until now only been investigated in rare occasions. E.g., US 2006/0100365 describes the reaction of free carbenes, such as imidazol-2-ylidenes and imidazolin-2-ylidenes, with ε-caprolactam. In this known technical proposal for the polymerization of ε-caprolactam, the unprotected carbene and monomers are heated to 200 °C for 60 min to provide a yield of 84%. However, a disadvantage of this process is that the product has a quite unfavorable ratio of carbene to ε-caprolactam of only 1:30, so that a large amount of carbene catalyst was necessary for the conversion. Other carbenes, which have been investigated, suffered from significantly poorer yields.

Another attempt to polymerize polyamides such as ε-CLA using N-heterocylic carbenes is described in WO 2014/118076 A1. In this document, monomer/ initiator ratios between 110:1 to 300:1 provided yields in the range of 27 to 85% after reaction at temperatures in the range of 140 to 180°C for 45 min.

DE 10 2013 210424 A1 discloses the synthesis of polyamides from laurolactam initiated by (protected) N-heterocyclic carbenes. In the reactions in this document, the monomer/initiator ratio was from 100:1 to 200:1 and yields from 71 to 100% were obtained at a reaction temperature of 180°C after 45 min.

Despite of these advances in the art, there is still a need for a reliable catalyst system, which provides high yields of polyamides from lactam starting materials at low temperature or with high reaction rates in short curing times and high yields. Preferably, the reactions should be sufficiently tolerant, so that they can be executed in substance or in solution, under inert gas or under ambient conditions. In addition, it should advantageously be possible to store a mixture of the catalyst system and the monomer for a prolonged period of time, such as for weeks and even month, to provide a ready to use material, which at the site of the application can simply be activated by heating at the reaction temperature without losing activity.

The present application addresses these needs.

### Description of the invention

In a first aspect, the invention concerns a catalyst system comprising
(i) a lactam activator (A) with increased electron deficiency at the intracyclic carbonyl carbon or precursors for the in-situ formation thereof and/or a Lewis acid (L) and
(ii) an N-heterocyclic carbene having the general formula (I), (II), (III), (IV), (V), (VI) and/or (VII), wherein
   the formula (I) is 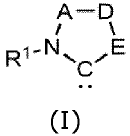
   wherein R¹ represents C₁-C₁₀ alkyl, C₂-C₁₀ alkenyl, C₃-C₁₂ cycloalkyl, C₆-C₁₀₀ polyoxyalkylene, C₅-C₁₀ aryl or C₅-C₁₀ heteroaryl;
   wherein A and D mutually independently represents a methylene moiety, CHR², and CR²R² or A and D together stand for a moiety from the series 1,2-phenylene, R²-C=N-, -N=N- or C₁-C₁₀ alkylen, C₂-C₁₀ alkenylen, C₃-C₁₂ cycloalkylen, C₆-C₁₀₀ polyoxyalkylene, C₅-C₁₀ aryl or C₅-C₁₀ hetaryl group-substituted 1,2-phenylene, CH=N, CH₂-NR²-, vinylene, -CH₂=CHR²-, -CHR²=CH₂-, -CHR²=CHR²-, the moiety R² mutually independently having the meaning given above for R¹;
   and wherein E represents oxygen, sulfur, -NR³'- or -PR³', R³' having the meaning given above for R¹;
   the formula (II) is 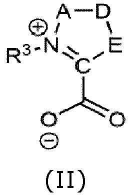
   wherein R³ has the meaning given above in formula (I) for R¹;
   wherein A and D have the meaning given above in formula (I) for A and D; wherein E has the meaning given above in formula (I) for E;
   and wherein O stands mutually independently for oxygen, sulfur or N-R¹⁰, preferably wherein O is oxygen, wherein R¹⁰ has the meaning given above in formula (I) for R¹;
   the formula (III) is

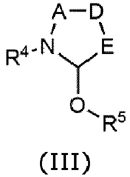

   wherein R⁴ has the meaning given above in formula (I) for R¹;
   wherein A and D have the meaning given above in formula (I) for A and D;
   wherein E has the meaning given above in formula (I) for E;
   and wherein R⁵ represents C₁-C₁₀ alkyl, C₅-C₁₂ cycloalkyl, C₆-C₁₀₀ polyoxyalkylene, C₅-C₁₀ aryl;
   the formula (IV) is

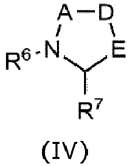

   wherein R⁶ has the meaning given above in formula (I) for R¹;
   wherein A and D have the meaning given above in formula (I) for A and D; wherein E has the meaning given above in formula (I) for E;
   and wherein R⁷ represents C₁-C₁₀ perfluoroalkyl, C₁-C₁₀ perchloroalkyl, partially fluorinated C₁-C₁₀ alkyl, partially chlorinated C₁-C₁₀ alkyl, perfluorinated C₅-C₁₀ aryl, partially fluorinated C₅-C₁₀ aryl, perchlorinated C₅-C₁₀ aryl, partially chlorinated C₅-C₁₀ aryl, preferably CF₃, CCl₃, pentafluorophenyl, tetrafluorophenyl moiety;
   the formula (V) is

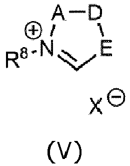

   wherein R⁸ has the meaning given above in formula (I) for R¹;
   wherein A and D have the meaning given above in formula (I) for A and D; wherein E has the meaning given above in formula (I) for E;
   and wherein X represents F⁻, Cl⁻, Br⁻, I, BF₄⁻, PF₆⁻, SbF₆⁻, HCO₃⁻, preferably wherein X is HCO₃⁻;
      and/or
   the formula (VI) is

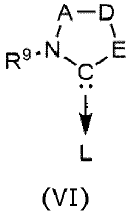

   wherein R⁹ has the meaning given above in formula (I) for R¹;
   wherein A and D have the meaning given above in formula (I) for A and D;
   wherein E has the meaning given above in formula (I) for E;
   and wherein L is a Lewis acid;
      and/or
   the formula (VII) is

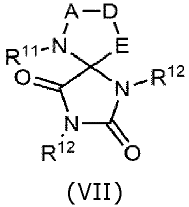

   wherein R¹¹ has the meaning given above in formula (I) for R¹;
   wherein A and D have the meaning given above in formula (I) for A and D;
   wherein E has the meaning given above in formula (I) for E;
   and wherein R¹² mutually independently represents the meaning given above for R¹, R⁷ or partially nitrated C₅-C₁₀ aryl;
   and wherein Y stands mutually independently for oxygen, sulfur or N-R¹⁰, preferably wherein Y is oxygen, wherein R¹⁰ has the meaning given above in formula (I) for R¹.

In other words, the inventive catalyst system comprises at least one of a lactam activator (A) with increased electron deficiency at the intracyclic carbonyl carbon, precursors for the in-situ formation of such lactam activator or a Lewis acid (L) and at least one N-heterocyclic carbene having the general formula (I), (II), (III), (IV), (V), (VI) or (VII).

The activator (A), as noted above, is a lactam with increased electron deficiency at the intracyclic carbonyl carbon, i.e. it has a substituent, which withdraws electron density from the intracyclic carbonyl carbon. Such substituents are preferably situated at the lactam nitrogen atom, as at this position they can be suitably be introduced by reaction with an isocyanate compound, acylation agent or phosphorylation agent. E.g. the lactam -NH**CO**-group on reaction with an isocyanate is reacted to a -N-(CO-NH-R)**CO**-, where the **CO**-group has reduced electron density. Similarly, upon reaction with an acylation agent or phosphorylation agent, the NH**CO**-group is reacted to a -N-(**CO**-R)CO-group or-N-(PO(OR)₂)**CO**-group with the same effect.

Suitable isocyanate compounds for this purpose include monoisocyanates, diisocyanates, and triisocyanates. Particularly suitable isocyanates are e.g. 1,6-hexamethylendiisocyanate (HDI), toluylendiisocynate (TDI) or methylendiphenyldiisocyanate (MDI) and 5-isocyanato-1-(isocyanatomethyl)-1,3,3-trimethylcyclohexane (IPDI), Cyclohexylisocyanat. A particularly suitable acylation agent is acetyl chloride.

Concerning the lactam, which forms the basis of the activator (A), the invention is not subject relevant restrictions, except that its lactam group should be sufficiently accessible for a reaction agent, which converts the lactam to a lactam with increased electron deficiency at the intracyclic carbonyl carbon. Preferred lactams for this purpose are lactams having 5 to 8, more preferably 6 or 7 ring forming atoms, such as 2-piperidinone or ε-caprolactam. In addition, it is preferred that the lactam is a lactam, which is also used in a later polymerisation with the inventive catalyst system.

Preferred precursors of the activator (A) are the respective lactams and isocyanate compounds, acylation agents and phosphorylation agents as indicated above.

Particularly preferred activators (A) include N,N'-(hexane-1,6-diyl)bis(2-oxoazepane-1-carboxamide) (activated lactam derived from ε-caprolactam and HDI), N,N'-(methylenebis(4,1-phenylene))bis(2-oxoazepane-1-carboxamide) (activated lactam derived from ε-caprolactam and MDI), N,N'-(4-methyl-1,3-phenylene)bis(2-oxoazepane-1-carboxamide) (activated lactam derived from ε-caprolactam and MDI), the reaction product of 5-isocyanato-1-(isocyanatomethyl)-1,3,3-trimethylcyclohexane and ε-caprolactam and/or N-acyllactams such as in particular 1-acetylazepan-2-one.

The Lewis acid for use in the catalyst system of the invention is a "Lewis acid" according to the definition of IUPAC as a molecular entity (and a corresponding chemical species), that is an electron-pair acceptor and therefore able to react with a Lewis base to form a Lewis adduct, by sharing the electron pair furnished by the Lewis base. Hence, the Lewis acid catalyst may be a metal ion or a metal ion complex within this definition.

The corresponding Lewis base that accompanies the Lewis acid catalyst is preferably of low nucleophilicity. Examples include halides such as bromide or iodide, a weak or non-coordinating anions such as tetraphenylborate, hexafluorophosphate, triflate (trifluoromethanesulfonate) and tosylate (p-tolylsulfonate). Particularly preferred nucleophiles are characterized by a low nucleophilicity with a value on the CH₃I scale (J. Am. Chem. Soc., 1977, 99, 24, 7968) of ≥ 2.0, more preferred of ≥ 4.0.

In the practice of the invention, the Lewis acid preferably comprises a metal ion or a metal ion complex selected from the group comprising
Li(I), Na(I), K(I), Rb(I), Cs(I), Ag(I), Au(I),
Mg(II), Ca(II), Sr(II), Ba(II), Dy(II), Yb(II), Cu(II), Zn(II), V(II), Mo(II), Mn(II), Fe(II), Co(II) Ni(II), Pd(II), Pt(II), Ge(II), Sn(II),
Sc(III), Y(III), La(III), Ce(III), Pr(III), Nd(III), Sm(III), Eu(III), Gd(III), Tb(III), Dy(III), Ho(III), Er(III), Tm(III), Yb(III), Lu(III), Hf(III), Nb(III), Ta(III), Cr(III), Ru(III), Os(III), Rh(III), Ir(III), AI(III), Ga(III), In(III), TI(III), Ge(III),
Ce(IV), Ti(IV), Zr(IV), Hf(IV), Nb(IV), Mo(IV), W(IV), Ir(IV), Pt(IV), Sn(IV), Pb(IV),
Nb(V), Ta(V), Sb(V), Bi(V),
Mo(VI), W(VI).

In the metal ion complex, an appropriately chosen ligand, such as phosphine, phosphite, arsenite, alkyl, acetylacetonate may be coordinated to the vacant coordination sites at the metal, whereby at least one coordination site remains available for binding of a Lewis base. The coordination site for binding of a Lewis base may also be created in the reaction mixture, e.g., by dissociation of a weakly coordinating ligand.

Preferred counter-anions in the invention are anions, which are weakly coordinating to the metal ion or metal ion complex.

Examples for preferred anions are selected from the group comprising chloride, bromide, iodide, tetrafluoroborate, RCO₂-, ROSO₃-, RSO₃-, [BR₄]-, [AlR₄]-, [GaR₄]-, [InR₄]-, wherein R represents hydrogen, a linear or branched, optionally heteroatom-including C₁- to C₂₂-alkyl residue, a linear or branched, mono- or polysubstituted, optionally heteroatom-including C₁- to C₂₂-alkenyl residue, a mono- or polysubstituted, optionally heteroatom-including C₆- to C₁₈-aryl residue, a saturated or unsaturated, optionally heteroatom-including 4- to 7-membered ring or polycyclic system, and wherein each R can be different. Examples of particularly preferred RSO₃- and ROSO₃-residues are tosylate (p-tolylsulfonate) or triflate (trifluoromethanesulfonate). From the aspect of costs, halides such as chloride, bromide and iodide are preferred, wherefrom chloride is most preferred.

In a particularly preferred embodiment, the metal ion in the Lewis acid is a small metal ion (i.e. of a metal with an order number of 30 or less in the periodic system, preferably with an order number of 13 or less). In another preferred embodiment, the metal ion in the Lewis acid has at least two and preferably at least three positive charges such as samarium (Sm). Especially preferred are small alkali or earth alkaline metal ions such as Li, Na, Mg and Ca, wherefrom Li and Mg are most preferred.

Especially preferred Lewis acids for use in the inventive catalyst system include LiCl, LiBr, LiI, MgCl₂, MgBr₂, MgI₂, Mg(OAc)₂, SmI₃, even more preferred is LiCl, LiBr and MgCl₂, and most preferred is MgCl₂.

In another embodiment, the Lewis acidic in the inventive catalyst system is combined with a bromide, iodide or RSO₄⁻ and/or RSO₃⁻ salt as co-catalyst, wherein R is an organic residue with the meaning as given above.

If the inventive catalyst system only contains an N-heterocyclic carbene of the formula (I) and a Lewis acid, the system includes the Lewis acid in more than a stoichiometric amount relative to the N-heterocyclic carbene of the formula (I).

The N-heterocyclic carbene in the inventive catalyst system is either a free N-heterocyclic carbene (as in the formula (I)) or N-heterocyclic carbene adduct with CO₂ (formula (II)), an alcohol (formula (III)), a halogenated organic compound (formula (IV)), an azolium hydrogencarbonate or azolium cation with another anion (formula V), an N-heterocyclic carbene Lewis acid adduct (formula VI) or an N-heterocyclic carbene adduct with an isocyanate compound (formula (VII)).

In these compounds, it is preferred that the residues R¹, R², R³, R⁴, R⁵, R⁶, R⁸, R⁹, R¹⁰, R¹¹ and R¹² are mutually independent and represent an alkyl group, preferably selected from methyl, ethyl, n-propyl, isopropyl, tert-butyl, neopentyl, isoamyl, cyclohexyl, or an aryl group, which is preferably selected from phenyl, 2,6-dimethylphenyl, 2,6-diisopropylphenyl or mesityl. In these compounds R⁷ represents CF₃, CCl₃, pentafluorophenyl, or tetrafluorophenyl, and the moiety -A-D- is selected from ethylene, vinylene, propylene of -CH=N-. Accordingly, the C-N-A-D-E-ring in the N-heterocyclic carbene is preferably a five of six membered ring with at least one, and preferably at least two nitrogen atoms. In addition, or in alternative, the C-N-A-D-E-ring is preferably an imidazolidine, an imidazoline or a triazol.

In another preferred embodiment, or in addition thereto, R¹, R², R³, R⁴, R⁵, R⁶, R⁸, R⁹, R¹⁰, R¹¹ and R¹² are mutually independent and represent methyl, isopropyl, phenyl or mesityl.

Particular preferred N-heterocyclic carbene-CO₂ adducts of the formula II for use in the inventive catalyst system are compounds having the formulae (II-1), (II-2), (II-3), (11-4), (II-5) and (II-6)

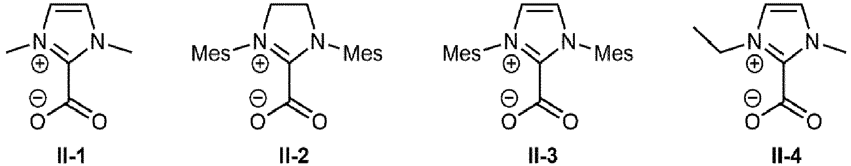

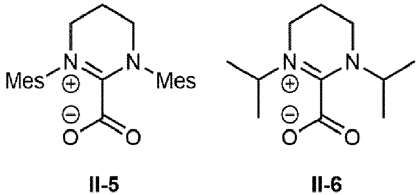

Suitable methods for the preparation of such compounds are e.g. described in Chem. Commun., 2003, 28-29, Chem. Eur. J., 2009, 15, 3103-3109 or Eur. J. Inorg. Chem., 2009, 1970-1976 or can be adapted from these references.

Particular preferred N-heterocyclic carbene-alcohol adducts of the formula III for use in the inventive catalyst system are compounds having the formulae (III-1) or (III-2)

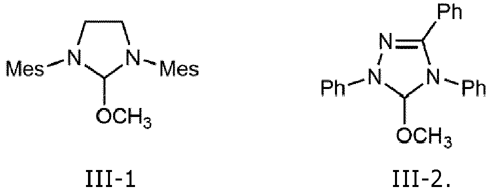

Suitable methods for the preparation of such compounds are e.g. described in J. Am. Chem. Soc., 2005, 127, 25, 9079-9084; or Angew. Chem. Int. Ed. Engl., 1995, 34, 9, 1021-1023.

Particular preferred N-heterocyclic carbene-adducts with halogenated organic compounds of the formula IV for use in the inventive catalyst system are compounds having the formulae (IV-1), (IV-2), (IV-3) or (IV-4)

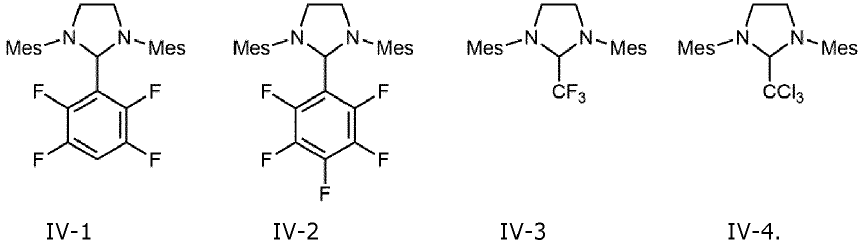

Suitable methods for the preparation of such compounds are e.g. described in Helv. Chim. Acta, 1999, 82, 12, 2348-2364 or Chem. Eur. J., 2004, 10, 4073-4079.

A particular preferred azolium hydrogen carbonate of an N-heterocyclic carbene of the formula (V) for use in the inventive catalyst system is a compound having the formula (V-1) 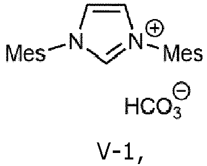 the preparation of which is described in J. Am. Chem. Soc., 2012, 134, 6776-6784.

Particular preferred adducts of an N-heterocyclic carbene with a Lewis acid of the formula (VI) for use in the inventive catalyst system are compounds having the formulae (VI-1) to (VI-10) as shown below.

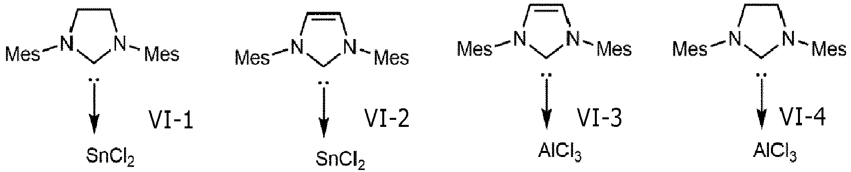

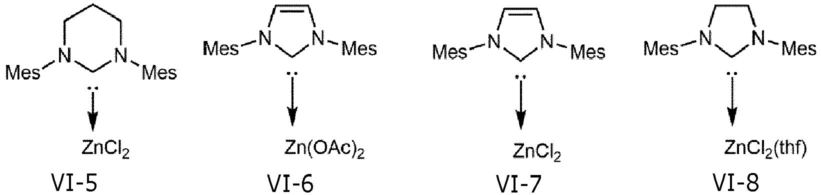

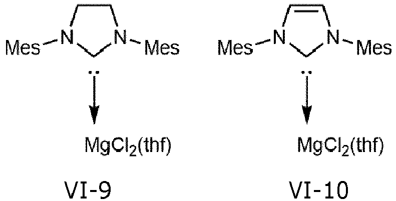

Suitable methods for the preparation of such compounds are e.g. described in Chem. Eur. J., 2009, 15, 3103-3109; Eur. J. Inorg. Chem., 2009, 1970-1976 or Polym. Chem., 2013, 4, 4172-4181. In the above "thf" indicates tetrahydrofurane, which is coordinated to the complex.

Finally, preferred adducts of an N-heterocyclic carbene with an isocyanate or isothiocyanate compound of the formula (VII) for use in the inventive catalyst system are compounds having the formulae (VII-1) to (VII-6) as shown below

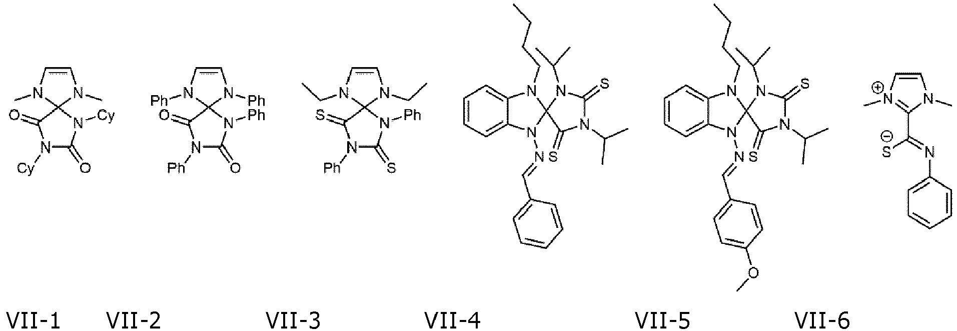

Suitable methods for the preparation of such compounds are e.g. described in J. Am. Chem. Soc., 1965, 87, 12, 2776-2777; Synthesis, 1970, 301-302; J. Org. Chem., 2011, 76, 301-304; J. Org. Chem., 2011, 76, 4082-4087; RCS Advances, 2013, 3, 1669-1672.

In a preferred embodiment, the inventive catalyst system comprises (i) a lactam activator (A) with increased electron deficiency at the intracyclic carbonyl carbon or precursors for the in-situ formation thereof and optionally a Lewis acid (L) and (ii) an N-heterocyclic carbene having the general formula (I), (II), (III), (IV), (V), (VI) and/or (VII). In a particularly preferred embodiment, the inventive catalyst system comprises both a lactam activator (A) or precursors for the in-situ formation thereof as defined above and a Lewis acid (L).

Concerning the ratio of the respective components, the inventive catalyst system is not subject to particular restrictions, as long as the catalyst system comprises at least one of the compounds in (i) and an N-heterocyclic carbene according to (ii). E.g. the molar ratio of the N-heterocyclic carbene to the Lewis acid can be from 1/20 until 20/1, preferably from 1/5 to 5/1, and more preferably from 1/2 to 2/1. For catalyst systems comprising N-heterocyclic carbene, activator (A) and Lewis acid (L) it has been found that a particularly suitable activity for the polymerization of lactams is provided with a ratio of the N-heterocyclic carbene/activator (A)/ Lewis acid (L) of from 5/1/1 to 1/5/1 or to 1/1/5 (i.e. one of the compounds may be present in an excess of up to fivefold over the other compounds); particularly preferred ratios are from 2/1/1 to 1/2/1 or to 1/1/2.

In another aspect, the present invention concerns a process for the production of a polyamide comprising reacting the respective polymer precursors with a catalyst system as specified above. In a preferred embodiment of this aspect, the process is for the production of poly(ε-caprolactam) or polylaurolactam and/or mixtures/mixed polymers thereof.

Lactams, which are used in the process as a precursor of the polyamides are cyclic structures of the general formula 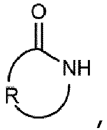 wherein preferably R is C₁-C₃₀ alkylen, C₂-C₃₀ alkenylen, C₃-C₁₂ cycloalkylen, C₂ to C₈-oxyalkylene, e.g. -CH₂-O-CH₂- or -CH₂-O-(CH₂)₂-, C₆-C₁₀₀ polyoxyalkylene, C₅-C₁₀ aryl or C₅-C₁₀ heteroarylene. Particularly preferred lactam for use in the process are ε-caprolactam and laurolactam.

The inventive catalyst system has the particular advantage that monomers for the production of a polyamide and the catalyst system can be mixed to provide a storage stable mixture, as long as the mixture is not exposed to temperatures where the polymerization is initiated (i.e. in these mixtures the catalyst system is a latent catalyst system). Thus, in a preferred aspect of the inventive process, the process comprises a step of homogenizing the catalyst system and the respective polymer precursors in a melt and optionally storing the mixture. The mixture can be stored in a molten state, or can be stored as a solid, wherefrom storage in a molten state is preferred.

Concerning the ratio of N-heterocyclic carbene in the catalyst system to monomer, the inventive process is not subject to relevant restrictions, except that the amount of the N-heterocyclic carbene should be high enough to provide the desired reaction speed. However, the N-heterocyclic carbene will regularly be present in a molar ratio, which is lower than that of the monomer. Particularly preferred molar ratios of N-heterocyclic carbene in the catalyst system to monomer are from 1/50 1 to 1/15000, especially from 1/100 to 1/10000, and even more preferably from 1/200 to 1/2500.

For the temperature, at which the polymerization is performed, it has been found that a suitable reaction speed can be provided in a temperature range of from 100°C to 300°C. A particularly suitable balance of reaction speed and energy input is provided at a range from 120°C to 250°C, and especially from 140°C to 220°C.

Further, in an advantageous embodiment, the inventive process can be executed in a reaction-injection-molding process (RIM) or a resin-transfer-molding process (RTM). Alternatively, or in addition, the inventive process can involve the injection of a mixture of the respective polymer precursors and the catalyst system into a mould and the initiation of the polymerization by an increase of the temperature.

For the modification of the product obtained by the process, it is possible to incorporate further additives or constituents into the polymerization mixture, such as fillers or reinforcing structures. Preferred fillers include e.g. SiO₂, Al₂O₃, TiO₂ or ZrO₂, and corresponding organic surface modified metal oxides. A possible reinforcing structures, which can be incorporated into the polymerization mixture, is e.g. a textile web.

In a yet further aspect, the invention concerns a process for the production of a polyamide, which comprises reacting the respective polymer precursors with a compound of the formula (VII) as defined above. In contrast to the above process, this process does not involve the additional presence of either of an activator (A) (or precursors thereof) and a Lewis acid (L).

In a yet further aspect, the invention concerns a polymerisation system for the production of a polyamide comprising a mixture of a cyclic amide, and a catalyst system as described above or a compound of the formula (VII) as defined above. For preferred embodiments of this polymerisation system, and preferred ingredients therein, reference is made to the embodiments, which have been indicated as preferred in connection with the description of the inventive catalyst systems and polymerisation processes above; these embodiments are likewise preferred for the polymerisation system.

In a yet further aspect, the invention concerns a compound of the formula (VII) as defined above, which is an adduct of an N-heterocyclic carbene with an isocyanate compound (i.e. wherein Y is O), in particular an adduct of the formula (VII-1) as defined above.

In a yet further aspect, the invention concerns a polyamide, which is prepared or preparable according to a processes as described above. Particularly preferred inventive polyamides will have a number average molecular weight Mₙ of ≥ 2000 g/mol to 1000000 g/mol, more preferably 5000 g/mol to 800000 g/mol, and even more preferably 5000 g/mol to 500000 g/mol. The respective molecular weight can conventionally be determined by GPC with appropriate standards such as polystyrene.

For the above polyamide, it is evident that to be distinguishable from polyamides prepared form other methods, it will regularly comprise some traces of the inventive catalyst system or a catalyst of formula (VII), where the amount of the catalyst is lower than the polyamide product directly obtained in the polymerisation. E.g. the polyamide can comprise less than 10 ppm, preferably less than 5 ppm and even more preferably less than 2 ppm of the N-heterocyclic carbenes (especially, when the -heterocyclic carbenes is a carbene according to the formula (IV), (V)of (VII)) in the catalyst system and optionally can comprises less than 10 ppm, preferably less than 5 ppm and even more preferably less than 2 ppm of the Lewis acid. The minimum amount of these compounds is regularly above their detection limit, but in some circumstances will be equal to or more than 0.1 ppm or even equal to or more than 0.5 ppm.

In the following, the invention will further be illustrated by means of examples, which however should not be construed as in any way limiting to the scope of the invention.

### Examples:

In the below tables, the abbreviations have the following meaning:

| | |
|---|---|
| 5u-Me-CO₂ | dimethylimidazolium-2-carboxylate |
| 5u-Me-(OCN-C_{y})₂ | dimethylimidazolium-adduct with two cylohexylisocyanate units |
| CLA-Ac | 1-acetylazepane-2-one, from Sigma Aldrich |
| CLA | ε-caprolactam, from Brüggemann GmbH und Co. KG |
| LLA | laurolactam, from Sigma Aldrich |
| C20P | (hexane-1,6-diyl)bis(2-oxoazepane-1-carboxamide), BRUGGOLEN® C20P |
| C25 | N,N'-(methylenebis(4,1-phenylene))bis(2-oxoazepane-1-carboxamide), BRUGGOLEN® C25 |
| MgCl₂ | from Sigma Aldrich |
| LiCl | from Grüssing |
| formic acid | from Acros Organics |
| acetone | from Friedrich Scharr |

In the below, all sample preparations were executed under inert gas atmosphere. Polymerizations reactions were done under inert gas conditions or under ambient conditions. Glassware was heated in an oven before use.

CLA, laurolactam, C20P, C25, MgCL₂, LiCl, formic acid and acetone were used as received. 1-Acetylazepan-2-one, prior to use, was dried, distilled and degassed. Cyclohexylisocyanate was distilled and degassed prior to use. The N-heterocyclic carbenes were synthesized according to the literature or with minor changes *(*Chem. Commun., 2003, 28-29; Organometallics, 2007, 26, 6042-6049; Chem, Eur. J., 2009, 15, 3103-3109; Eur. Polym. J., 2017, 95, 766-774).

The work-up in all of the below Examples was performed as follows:
The respective reaction product was dissolved in formic acid at room temperature and then poured into acetone. If a polymer had formed it precipitated, and the precipitate was separated by centrifuging. The supernatant solution was discarded. The residual solid was dried under reduced pressure at temperatures between 15 and 100°C. Subsequently the yield was determined. Otherwise noted.

### Example 1 (In situ-homogenization):

The compounds as indicated in the following tables 1 to 3 were weighed in a glass vial, which was equipped with a magnetic stir bar. The mixture was heated to a temperature of 200°C (table 1) and 180°C (tables 2 and 3, respectively) for 60 min to execute the reaction. The yields of the reaction are also provided in the following tables 1 to 3.

**Table 1:**

| 5u-Me-CO₂^{a} | CLA-Ac^{a} | MgCl₂^{a} | LiCl^{a} | CLA^{a} | Yield [%] |
|---|---|---|---|---|---|
| - | - | - | 1 | 100 | <1 |
| - | - | 1 | - | 100 | <1 |
| - | 1 | - | - | 100 | <1 |
| 1 | - | - | - | 100 | 17 |
| 1 | - | - | 1 | 100 | 33 |
| 1 | - | 1 | - | 100 | 37 |
| 1 | 1 | - | - | 100 | 30 |
| 1 | 1 | - | 1 | 100 | 72 |
| 1 | 1 | 1 | - | 100 | >96 |

| | | | | | |
|---|---|---|---|---|---|
| ^{a} amount of a certain compound in equivalents. | | | | | |

**Table 2:**

| 5u-Me-CO₂^{a} | CLA-Ac^{a} | MgCl₂^{a} | CLA^{a} | LLA^{a} | Yield [%] |
|---|---|---|---|---|---|
| 1 | 1 | 1 | 500 | - | >96 |
| 1 | 1 | 1 | 375 | 125 | 68 |
| 1 | 1 | 1 | 250 | 250 | 53 |
| 1 | 1 | 1 | 125 | 375 | 42 |
| 1 | 1 | 1 | - | 500 | 0 |

| | | | | | |
|---|---|---|---|---|---|
| ^{a} amount of a certain compound in equivalents. | | | | | |

**Table 3:**

| 5u-Me-(OCN-Cy)₂^{a} | CLA-Ac^{a,c} | MgCl₂^{a} | CLA^{a} | solidification time [min] | Yield [%] |
|---|---|---|---|---|---|
| 1 | 1 | 1 | 250 | 3 | ≥ 96 |
| 1 | - | 1 | 250 | 9 | ≥ 96 |
| 1 | - | - | 250 | 7 | ≥ 96 |

| | | | | | |
|---|---|---|---|---|---|
| ^{a} amount of a certain compound in equivalents. | | | | | |

### Example 2 (Pre-homogenization - storage as solid)

All substances, i.e. the N-heterocyclic carbene, activator and monomer were weighed into a glass vial, which was equipped with a magnetic stir bar. The mixture was stirred and heated to 75 to 90°C for 1 to 12 h. In due course a solution formed. The solution was either reacted directly or cooled to room temperature upon which the mixture solidified and was stored and reacted later.

**Table 4: Solidification times and yields of polymerizations of CLA at different temperatures.**

| eq. CLA | 100 | | 200 | | 300 | | 400 | |
|---|---|---|---|---|---|---|---|---|
| T(°C) | t^{a} | yield (%) | t^{a} | yield (%) | t^{a} | yield (%) | t^{a} | yield (%) |
| 140 | - | - | 12 | >96 | - | - | - | - |
| 160 | 6 | >96 | 7 | > 96 | 12 | >96 | 10 | >96 |
| 180 | 3 | >96 | 4 | > 96 | 6 | >96 | 6 | >96 |
| 200 | 2 | >96 | 3 | >96 | 4 | > 96 | 4 | > 96 |

| | | | | | | | | |
|---|---|---|---|---|---|---|---|---|
| ^{a} solidification times in min. | | | | | | | | |

In the header of the above table 100 means: 100 eq. CLA, 1 eq. 5u-Me-CO₂, 1 eq. CLA-Ac and 1 eq. MgCl₂; 200: means 200 eq. CLA, 1 eq. 5u-Me-CO₂, 1 eq. CLA-Ac and 1 eq. MgCl₂; 300 means: 300 eq. CLA, 1 eq. 5u-Me-CO₂, 1 eq. CLA-Ac and 1 eq. MgCl₂; and 400 means: 400 eq. CLA, 1 eq. 5u-Me-CO₂, 1 eq. CLA-Ac and 1 eq. MgCl₂.

**Table 5: Solidification times and polymerization yields. In these experiments, the isocyanate adduct of the NHC was prepared in situ.**

| 5u-Me-CO₂^{a} | Cy-NCO^{a} | CLA-Ac^{a} | MgCl₂^{a} | CLA^{a} | T (°C) | t^{b} | Yield (%) |
|---|---|---|---|---|---|---|---|
| 1 | 2 | 1 | 1 | 250 | 180 | 3 | >96 |
| 1 | 2 | - | 1 | 250 | 180 | 12 | 93 |
| 1 | 2 | - | - | 250 | 180 | 18 | 72 |

| | | | | | | | |
|---|---|---|---|---|---|---|---|
| ^{a} amount of a certain compound in equivalents, ^{b} solidification time in min. Reaction time was 60 min. | | | | | | | |

**Table 6: Yields of polymerizations of CLA at different temperatures.**

| 5u-Me-CO₂^{a} | C20P^{a} | C25^{a} | MgCl₂^{a} | CLA^{a} | T (°C) | Yield (%) |
|---|---|---|---|---|---|---|
| 1 | 1 | - | 1 | 300 | 180 | > 96 |
| 1 | 1 | - | 1 | 300 | 200 | >96 |
| 2 | 1 | - | 2 | 300 | 180 | > 96 |
| 2 | 1 | - | 2 | 300 | 200 | >96 |
| 2 | 1 | - | 2 | 600 | 180 | > 96 |
| 2 | 1 | - | 2 | 600 | 200 | >96 |
| 1 | - | 1 | 1 | 300 | 180 | > 96 |
| 1 | - | 1 | 1 | 300 | 200 | >96 |
| 2 | - | 1 | 2 | 300 | 180 | > 96 |
| 2 | - | 1 | 2 | 300 | 200 | >96 |
| 2 | - | 1 | 2 | 600 | 180 | > 96 |
| 2 | - | 1 | 2 | 600 | 200 | >96 |

| | | | | | | |
|---|---|---|---|---|---|---|
| ^{a} amount of a certain compound in equivalents. | | | | | | |

In the above table, the reaction was time 60 min. Due to insolubility of the product in formic acid the material was extracted with propan-2-one to remove residual monomer, initiator and activator and dried under reduced pressure before the yield was determined.

### Example 3: Pre-homogenization - storage as solid -diluted with additional monomer

All substances, i.e. the N-heterocyclic carbene, activator and monomer were weighed in a glass vial, which was equipped with a magnetic stir bar. The mixture was stirred and heated to 75 to 90°C for 1 to 12 h. In due course a solution formed. The solution was either reacted directly or cooled to room temperature upon which the mixture solidified and was stored and reacted later. The mixtures were diluted with further monomer before the polymerization reaction (at 200°C for 60 min). In each case, a mixture of the equivalents of CLA as indicated in table 7 below with 1 eq. 5u-Me-CO₂, 1 eq. CLA-Ac and 1 eq. MgCl₂ was reacted. If more than 100 equivalents of CLA were used, the reaction mixture was prepared from a mixture with 100 equivalents of CLA via the addition of further monomer.

**Table 7: Yields of polymerization**

| 5u-Me-CO₂^{a} | CLA-Ac^{a} | MgCl₂^{a} | CLA^{a} | Yield (%) |
|---|---|---|---|---|
| 1 | 1 | 1 | 50 | >96 |
| 1 | 1 | 1 | 100 | >96 |
| 1 | 1 | 1 | 200 | >96 |
| 1 | 1 | 1 | 250 | >96 |
| 1 | 1 | 1 | 300 | > 96 |
| 1 | 1 | 1 | 400 | >96 |
| 1 | 1 | 1 | 500 | >96 |
| 1 | 1 | 1 | 1000 | >96 |

| | | | | |
|---|---|---|---|---|
| ^{a} amount of a certain compound in equivalents. | | | | |

### Example 4: Pre-homogenization - storage as solid - polymerized under ambient atmosphere

All substances, i.e. the N-heterocyclic carbene, activator and monomer were weighed in a glass vial, which was equipped with a magnetic stir bar. The mixture was stirred and heated to 75 to 90°C for 1 to 12 h. In due course, a solution formed. The solution was either reacted directly (Approach 1) or cooled to room temperature were the mixture solidified and was stored and reacted later under ambient atmosphere (approach 2). The respective mixtures each contained 100 eq. CLA, 1 eq. 5u-Me-CO₂, 1 eq. CLA-Ac and 1 eq. MgCl₂.

**Table 8: Solidification times and yields of the polymerizations**

| | Approach 1 | | Approach 2 | |
|---|---|---|---|---|
| T^{a} | t^{b} | yield^{c} | t^{b} | yield^{c} |
| 160 | 6 | > 96 | 9 | >96 |
| 180 | 3 | >96 | 5 | > 96 |
| 200 | 2 | >96 | 3 | >96 |

| | | | | |
|---|---|---|---|---|
| ^{a} reaction temperature in °C, ^{b} solidification times in min, ^{c} yields in %. | | | | |

### Example 5: Pre-homogenization - storage in melt

All substances, i.e. the N-heterocyclic carbene, activator and monomer were weighed in a glass vial, which was equipped with a magnetic stir bar. The mixture was stirred and heated to 75 to 90°C for 1 to 12 h. In due course a solution formed. The solution was stored as melt at 75-90°C for 2h (Approach 3) and 5d (Approach 4) and reacted later. The respective mixtures each contained 200 eq. CLA, 1 eq. 5u-Me-CO₂, 1 eq. CLA-Ac and 1 eq. MgCl₂.

**Table 9: Solidification times and yields of the polymerizations**

| | Approach 3 | | Approach 4 | |
|---|---|---|---|---|
| T(°C) | t^{a} | yield (%) | t^{a} | Yield (%) |
| 160 | 7 | > 96 | 7 | > 96 |
| 180 | 4 | > 96 | 4 | > 96 |
| 200 | 3 | >96 | 4 | > 96 |

| | | | | |
|---|---|---|---|---|
| ^{a} solidification times in min. | | | | |

### Example 6: Microwave assisted heating

All substances, i.e. the N-heterocyclic carbene, activator and monomer were weighed in a glass vial. The mixture was heated to reaction temperature (180°C for 45 min) using a microwave.

**Table 10: Microwave assisted polymerization.**

| 5u-Me-CO₂^{a} | CLA-Ac^{a} | MgCl₂^{a} | CLA^{a} | Yield (%) |
|---|---|---|---|---|
| 1 | 1 | 1 | 100 | >96 |

| | | | | |
|---|---|---|---|---|
| ^{a} amount of a certain compound in equivalents. | | | | |

## Claims

1. A catalyst system comprising
(i) a lactam activator (A) with increased electron deficiency at the intracyclic carbonyl carbon or precursors for the in-situ formation thereof and/or a Lewis acid (L) and
(ii) an N-heterocyclic carbene having the general formula (I), (II), (III), (IV), (V), (VI) and/or (VII), wherein
the formula (I) is 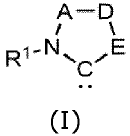
wherein R¹ represents C₁-C₁₀ alkyl, C₂-C₁₀ alkenyl, C₃-C₁₂ cycloalkyl, C₆-C₁₀₀ polyoxyalkylene, C₅-C₁₀ aryl or C₅-C₁₀ heteroaryl;
wherein A and D mutually independently represents a methylene moiety, CHR², and CR²R² or A and D together stand for a moiety from the series 1,2-phenylene, R²-C=N-, -N=N- or C₁-C₁₀ alkylen, C₂-C₁₀ alkenylen, C₃ C₁₂ cycloalkylen, C₆-C₁₀₀ polyoxyalkylene, C₅-C₁₀ aryl or C₅-C₁₀ hetaryl group-substituted 1,2-phenylene, CH=N, CH₂-NR²-, vinylene, -CH₂=CHR²-, -CHR²=CH₂-, -CHR²=CHR²-, wherein the moiety R² mutually independently has the meaning given above for R¹;
and wherein E represents oxygen, sulfur, -NR^{3'}- or -PR^{3'}, R^{3'} having the meaning given above for R¹;
the formula (II) is 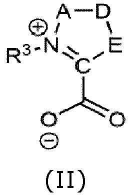
wherein R³ has the meaning given above in formula (I) for R¹;
wherein A and D have the meaning given above in formula (I) for A and D; wherein E has the meaning given above in formula (I) for E;
and wherein O stands mutually independently for oxygen, sulfur or N-R¹⁰, preferably wherein O is oxygen, wherein R¹⁰ has the meaning given above in formula (I) for R¹;
the formula (III) is 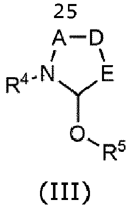
wherein R⁴ has the meaning given above in formula (I) for R¹;
wherein A and D have the meaning given above in formula (I) for A and D;
wherein E has the meaning given above in formula (I) for E;
and wherein R⁵ represents C₁-C₁₀ alkyl, C₅-C₁₂ cycloalkyl, C₆-C₁₀₀ polyoxyalkylene, C₅-C₁₀ aryl;
the formula (IV) is 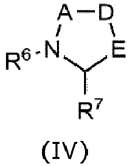
wherein R⁶ has the meaning given above in formula (I) for R¹;
wherein A and D have the meaning given above in formula (I) for A and D;
wherein E has the meaning given above in formula (I) for E;
and wherein R⁷ represents C₁-C₁₀ perfluoroalkyl, C₁-C₁₀ perchloroalkyl, partially fluorinated C₁-C₁₀ alkyl, partially chlorinated C₁-C₁₀ alkyl, perfluorinated C₅-C₁₀ aryl, partially fluorinated C₅-C₁₀ aryl, perchlorinated C₅-C₁₀ aryl, partially chlorinated C₅-C₁₀ aryl, preferably CF₃, CCl₃, pentafluorophenyl, tetrafluorophenyl moiety;
the formula (V) is 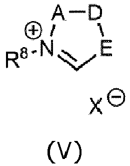
wherein R⁸ has the meaning given above in formula (I) for R¹;
wherein A and D have the meaning given above in formula (I) for A and D;
wherein E has the meaning given above in formula (I) for E;
and wherein X represents F⁻, Cl⁻, Br⁻, I, BF₄⁻, PF₆⁻, SbF₆⁻, HCO₃⁻, preferably
wherein X is HCO₃⁻;
and/or
the formula (VI) is 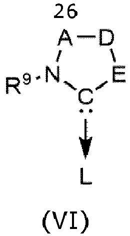
wherein R⁹ has the meaning given above in formula (I) for R¹;
wherein A and D have the meaning given above in formula (I) for A and D; wherein E has the meaning given above in formula (I) for E;
and wherein L is a Lewis acid;
and/or
the formula (VII) is 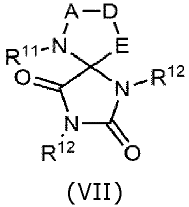
wherein R¹¹ has the meaning given above in formula (I) for R¹;
wherein A and D have the meaning given above in formula (I) for A and D;
wherein E has the meaning given above in formula (I) for E;
and wherein R¹² mutually independently has the meaning given above for R¹, R⁷ or partially nitrated C₅-C₁₀ aryl;
and wherein Y stands mutually independently for oxygen, sulfur or N-R¹⁰, preferably wherein Y is oxygen, wherein R¹⁰ has the meaning given above in formula (I) for R¹.

2. The catalyst system of claim 1, wherein the lactam activator (A) is the reaction product of a lactam, preferable with 5 to 8 and more preferably 6 or 7 ring forming atoms, and an isocyanate compound, an acylation agent or a phosphorylation agent or wherein the precursors for the in-situ formation of the lactam activator are a lactam and an isocyanate compound, an acylation agent or a phosphorylation agent.

3. Catalysts system according to claim 2, wherein the activator (A) is one or more compound(s) selected from the group of adduct(s) of lactams with isocyanates and N-acyllactams, preferably N,N'-(hexane-1,6-diyl)bis(2-oxoazepane-1-carboxamide), N,N'-(methylenebis(4,1-phenylene))bis(2-oxoazepane-1-carboxamide), N,N'-(4-methyl-1,3-phenylene)bis(2-oxoazepane-1-carboxamide), 5-isocyanato-1-(isocyanatomethyl)-1,3,3-trimethylcyclohexane), N-cyclohexyl-2-oxoazepane-1-carboxamide and/or 1-acetylazepan-2-one, more preferably N,N'-(hexane-1,6-diy!)bis(2-oxoazepane-1-carboxamide), N,N'-(methylenebis(4,1-phenylene))bis(2-oxoazepane-1-carboxamide), N,N'-(4-methyl-1,3-phenylene)bis(2-oxoazepane-1-carboxamide), N-cyclohexyl-2-oxoazepane-1-carboxamide, 1-acetylazepan-2-one, and most preferably 1-acetylazepan-2-one.

4. Catalysts system according to any one of the preceding claims, wherein the Lewis acid (L) is one or more compound(s) selected from the group of lithium, magnesium of samarium halides, preferably from LiCl, LiBr, LiI, MgCl₂, MgBr₂, MgI₂, Mg(OAc)₂, SmI₃, more preferably from LiCI and MgCl₂, and most preferable wherein the Lewis acid is MgCl₂.

5. Catalysts system according to any one of the preceding claims, wherein moieties R¹, R², R³, R⁴, R⁵, R⁶, R⁸, R⁹, R¹⁰, R¹¹ and R¹² in the formulae (I), (II), (III), (IV), (V), (VI) and (VII) are mutually independent and represent methyl, ethyl, n-propyl, isopropyl, tert-butyl, neopentyl, isoamyl, cyclohexyl, phenyl, 2,6-dimethylphenyl, 2,6-diisopropylphenyl or mesityl moiety; R⁷ represents CF₃, CCl₃, pentafluorophenyl, or tetrafluorophenyl; and wherein the moieties A and D stand for an ethylene, vinylene or propylene moiety.

6. Catalysts system according to claim 5, wherein R¹, R², R³, R⁴, R⁵, R⁶, R⁸, R⁹, R¹⁰, R¹¹ and R¹² are mutually independent and represent methyl, isopropyl, phenyl or mesityl.

7. Catalysts according to claim 5, wherein the N-heterocyclic carbene is one or more compounds having the formulae (II-1), (II-2), (II-3), (II-4), (II-5) and (II-6)

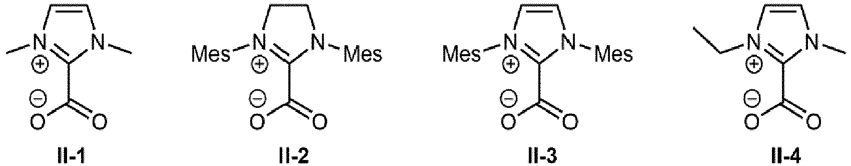

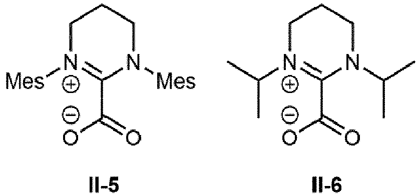

8. Catalysts system according to any one of the preceding claims, wherein the molar ratio of N-heterocyclic carbene to activator to Lewis acid is from 5/1/1 to 1/5/1 or to 1/1/5, preferably from 2/1/1 to 1/2/1 or to 1/1/2.

9. Process for the production of a polyamide, preferably poly(ε-caprolactam) or polylaurolactam and/or mixed polymers thereof, comprising reacting the respective polymer precursors with a catalyst system according to any one of claim 1 to 8.

10. Process according to claim 9, which involves a step of homogenizing the catalyst system and the respective polymer precursors in a melt and optionally storing the mixture, preferably in the molten state.

11. Process according to claim 9 or 10, wherein the molar ratio of N-heterocyclic carbene in the catalyst system to monomer is in the range of 1 to 50 up to 1 to 15000, preferably 1 to 100 up to 1 to 10000, and more preferably 1 to 200 up to 1 to 2500.

12. Process according to any one of claims 9 to 11, wherein the polymerisation reaction is executed at a temperature in the range of 100°C to 300°C, preferably in the range from 120°C to 250°C, and more preferably from 140°C to 220°C, and/or wherein the polymerization reaction is executed in a reaction-injection-molding process (RIM) or a resin-transfer-molding process (RTM), or wherein the process comprises the injection of a mixture of the respective polymer precursors and the catalyst system into a mould and the initiation of the polymerization by an increase of the temperature.

13. Process according to any one of claims 9 to 12, wherein the reaction mixture, which is subjected to polymerization further contains one or more fillers, preferably selected from SiO₂, Al₂O₃, TiO₂ or ZrO₂, and corresponding organic surface modified metal oxides, or reinforcing structures, preferably as textile webs.

14. Process for the production of a polyamide, preferably poly(ε-caprolactam) or polylaurolactam and/or mixtures thereof, comprising reacting the respective polymer precursors with a compound of the formula (VII) as defined in claim 1.

15. A compound of the formula (VII), 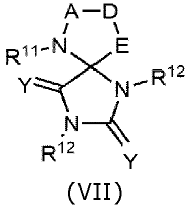 which is an adduct of an N-heterocyclic carbene with an isocyanate compound (R-NCO), preferably a compound of the formula (VII-1)

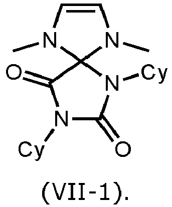

16. A polymerisation system for the production of a polyamide comprising a mixture of a cyclic amide, preferably ε-caprolactam and/or laurolactam, and a catalyst system according to any one of claims 1 to 8.

17. Polyamide, in particular having a number average molecular weight Mₙ of ≥ 2000 g/mol to 1000000 g/mol, more preferably 5000 g/mol to 800000 g/mol, and even more preferably 5000 g/mol to 500000 g/mol, which is prepared according to a process as described in any one of claim 9 to 15.
